# EUROPEAN PATENT APPLICATION

(11) **EP 4 772 859 A1**
(43) Date of publication of application: **08.07.2026**
(21) Application number: 24857821.3
(22) Date of filing: 06.05.2024
(51) Int. Cl.: G01N 21/27, A47L 11/40

(54) **WASTEWATER TESTING APPARATUS AND CLEANING DEVICE**

(30) Priority: 25.08.2023 CN 202322312599 U
(71) Applicant: Beijing Roborock Technology Co., Ltd., Beijing 102206 (CN)
(72) Inventor: YU, Luping, Beijing 102206 (CN)
(74) Representative: Winter, Brandl - Partnerschaft mbB
(86) International application number: PCT/CN2024/091236
(87) International publication number: WO 2025/044271

(57) **Abstract**

A wastewater testing apparatus and a cleaning device. The wastewater testing apparatus comprises: a housing (100) provided therein with an accommodating cavity, wherein the housing (100) is provided thereon with a water inlet (111), a water outlet (112), and a wire hole (113) which are communicated with the accommodating cavity; a testing tube (200) arranged in the accommodating cavity, wherein two ends of the testing tube (200) are respectively communicated with the water inlet (111) and the water outlet (112); and a testing assembly (300) arranged in the accommodating cavity, wherein the testing assembly (300) comprises a transmitter (310), a receiver (320), a circuit board (330) connected to the transmitter (310) and the receiver (320), and a wire harness connected to the circuit board (330), the transmitter (310) and the receiver (320) are arranged opposite to each other on two sides of the testing tube (200), and the wire harness extends to the outside of the housing (100) by means of the wire hole (113).

## Description

### CROSS-REFERENCE TO RELATED APPLICATION

The present disclosure claims priority to Chinese Patent Application No. 202322312599X filed on August 25, 2023, the content of which is incorporated herein by reference in its entirety.

### TECHNICAL FIELD

The present disclosure relates to the technical field of cleaning devices, and in particular, to a wastewater detection apparatus and a cleaning device.

### BACKGROUND ART

A cleaning device generates wastewater during cleaning, and the cleaning effectiveness can be determined by detecting the degree of contamination in the wastewater. In the related art, the arrangement of the wastewater detection apparatus in the cleaning device is relatively complex, making its application in the cleaning device quite inconvenient.

It should be noted that the information disclosed in the above background section is only used for the enhancement of understanding of the background of the present disclosure and, therefore, may include information that does not constitute the prior art known to those of ordinary skill in the art.

### SUMMARY OF THE INVENTION

The present disclosure is intended, at least to some extent, to solve the technical problem that the arrangement of a conventional wastewater detection apparatus in a cleaning device is relatively complex. To this end, the present disclosure provides a wastewater detection apparatus and a cleaning device.

According to an aspect of the embodiments of the present disclosure, a wastewater detection apparatus is provided. The wastewater detection apparatus includes:
a housing, the housing being provided with an accommodating cavity therein, and the housing being provided with a water inlet, a water outlet, and a wiring hole that are in communication with the accommodating cavity;
a detection tube, the detection tube being arranged in the accommodating cavity, and two ends of the detection tube being in communication with the water inlet and the water outlet, respectively; and
a detection assembly, the detection assembly being arranged in the accommodating cavity, and the detection assembly including a transmitter, a receiver, a circuit board connected to the transmitter and the receiver, and a wire harness connected to the circuit board, where the transmitter and the receiver are arranged oppositely on two sides of the detection tube, and the wire harness extends to outside of the housing through the wiring hole.

In some embodiments, the detection assembly is a light detection assembly, at least two opposite sides of the detection tube are provided with light-transmitting surfaces, and the transmitter and the receiver correspond to the light-transmitting surfaces.

In some embodiments, at least the light-transmitting surfaces of the detection tube are perpendicular to detection light emitted by the light detection assembly.

In some embodiments, the detection tube is manufactured from a light-transmitting material.

In some embodiments, the housing and the detection tube are formed by two-color injection molding, the housing is made of a light-shielding injection molding material, and the detection tube is made of a light-transmitting injection molding material; or, the housing includes a housing body and a light-shielding layer provided on the housing body, the housing body and the detection tube are integrally formed by injection molding, and the housing body and the detection tube are made of a light-transmitting injection molding material.

In some embodiments, the water inlet is provided with a water inlet connector, and the water outlet is provided with a water outlet connector.

In some embodiments, a connecting part is provided on an outer side of the housing, and the connecting part is configured for connection with a cleaning device.

In some embodiments, the housing includes a first shell and a second shell, and the second shell is fastened onto the first shell to enclose and form the accommodating cavity.

In some embodiments, the wiring hole is provided with a sealing rubber sleeve; a sealing gasket or a sealing ring is provided on one side of the second shell facing the first shell.

In some embodiments, a fixing part and/or a positioning part is provided on an inner side of the housing; the fixing part is configured to fix the circuit board, and the positioning part is configured to position the circuit board.

In some embodiments, the circuit board includes a circuit substrate, as well as a transmitting-end circuit module and a receiving-end circuit module that are arranged on the circuit substrate; the transmitting-end circuit module includes the transmitter, the receiving-end circuit module includes the receiver, and the transmitting-end circuit module and the receiving-end circuit module are arranged on the same circuit substrate or arranged respectively on two circuit substrates.

According to another aspect of the embodiments of the present disclosure, a cleaning device is provided. The cleaning device includes the wastewater detection apparatus described above.

The embodiments of the present disclosure have at least the following beneficial effects:
In the wastewater detection apparatus described above, the wastewater detection apparatus is connected in series to a wastewater pipeline via the water inlet and a wastewater outlet on the housing, such that the wastewater in the wastewater pipeline can be detected. The wastewater detection apparatus is simple to arrange and operate in the cleaning device, and is convenient in application. Meanwhile, the wastewater detection apparatus seals the detection tube and the detection assembly inside the accommodating cavity by means of the housing, forming a stable and clean detection environment in the accommodating cavity. This prevents dust and moisture from entering the accommodating cavity to contaminate the detection tube or interfere with the detection signals of the detection assembly, thereby ensuring the accuracy and stability of the wastewater detection apparatus. Furthermore, the detection tube and the detection assembly are protected, which prevents damage to the detection tube and the detection assembly, thereby prolonging the service life of the wastewater detection apparatus.

### BRIEF DESCRIPTION OF THE DRAWINGS

For a clearer illustration of the technical solutions in the embodiments of the present disclosure, the drawings required to be used in the description of the embodiments are briefly introduced below. It is apparent that the drawings in the description below are for some embodiments of the present disclosure, and for those of ordinary skill in the art, other drawings can be acquired according to these drawings without creative efforts.
FIG. 1 shows a schematic diagram of a three-dimensional structure of a wastewater detection apparatus according to an embodiment of the present disclosure;
FIG. 2 shows a schematic diagram of a three-dimensional structure of the wastewater detection apparatus of FIG. 1 from another angle of view;
FIG. 3 shows a schematic diagram of a three-dimensional structure of the wastewater detection apparatus of FIG. 1 with a housing cover removed;
FIG. 4 shows a schematic diagram of a three-dimensional structure of the wastewater detection apparatus of FIG. 3 with the housing cover removed from another angle of view;
FIG. 5 shows a front view of the wastewater detection apparatus of FIG. 3 with the housing cover removed;
FIG. 6 shows a schematic diagram of a three-dimensional structure of a shell of the wastewater detection apparatus of FIG. 1;
FIG. 7 shows a schematic diagram of a three-dimensional structure of the housing cover of the wastewater detection apparatus of FIG. 1; and
FIG. 8 shows a schematic diagram of a three-dimensional structure of the housing cover of the wastewater detection apparatus of FIG. 1 from another angle of view.

### Reference numerals:

100, housing; 110, first shell; 111, water inlet; 1111, water inlet connector; 112, water outlet; 1121, water outlet connector; 113, wiring hole; 1131, sealing rubber sleeve; 114, connecting part; 115, fixing part; 116, positioning part; 120, second shell; 121, sealing gasket; 122, connecting member; 123, connecting washer; 200, detection tube; 210, light-transmitting surface; 300, detection assembly; 310, transmitter; 320, receiver; 330, circuit board; 331, transmitting-end circuit module; 332, receiving-end circuit module; 333, fixing hole; 334, positioning hole.

### DETAILED DESCRIPTION

The technical solutions in embodiments of the present disclosure will be clearly and completely described below with reference to the drawings in the embodiments of the present disclosure. It is apparent that the described embodiments are merely some, but not all, of the embodiments of the present disclosure. Based on the embodiments in the present disclosure, all other embodiments obtained by those of ordinary skill in the art without creative efforts shall fall within the protection scope of the present disclosure.

Furthermore, the present disclosure may repeat reference numbers and/or reference letters in different examples. Such repetition is intended for simplicity and clarity rather than for indicating the relationship between various embodiments and/or arrangements discussed. Furthermore, the present disclosure provides examples of various specific processes and materials, but those of ordinary skill in the art will recognize the application of other processes and/or the use of other materials.

The present disclosure is described below in conjunction with the accompanying drawings and with reference to specific embodiments.

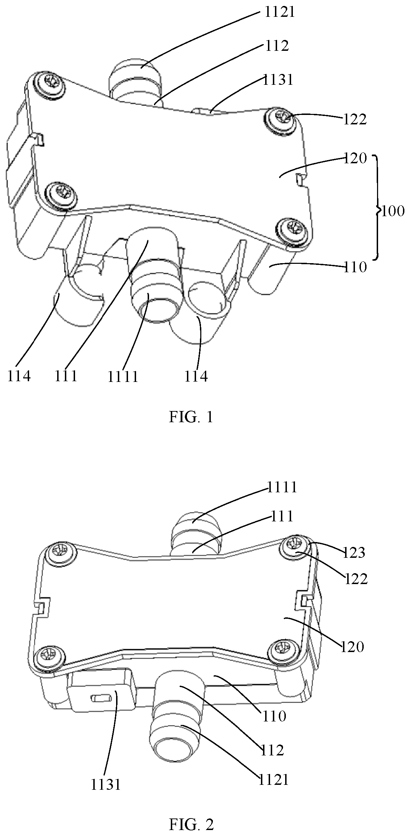

The embodiments of the present disclosure provide a wastewater detection apparatus. As shown in FIGs. 1 to 8, the wastewater detection apparatus includes a housing 100, a detection tube 200, and a detection assembly 300. The housing 100 is provided with an accommodating cavity therein. The housing 100 is provided with a water inlet 111, a water outlet 112, and a wiring hole 113 that are in communication with the accommodating cavity. The detection tube 200 is arranged in the accommodating cavity, with both ends of the detection tube 200 in communication with the water inlet 111 and the water outlet 112, respectively. The detection assembly 300 is arranged in the accommodating cavity. The detection assembly 300 includes a transmitter 310, a receiver 320, a circuit board 330 connected to the transmitter 310 and the receiver 320, and a wire harness connected to the circuit board 330. The transmitter 310 and the receiver 320 are oppositely arranged on both sides of the detection tube 200. The wire harness extends to the outside of the housing 100 through the wiring hole 113.

In the wastewater detection apparatus of the present disclosure, as shown in FIGs. 1 to 8, the wastewater detection apparatus is connected in series to a wastewater pipeline via the water inlet 111 and a wastewater outlet on the housing 100, such that the wastewater in the wastewater pipeline can be detected. The wastewater detection apparatus is simple to arrange and operate in the cleaning device, and is convenient in application. Meanwhile, the wastewater detection apparatus seals the detection tube 200 and the detection assembly 300 inside the accommodating cavity by means of the housing 100, forming a stable and clean detection environment in the accommodating cavity. This prevents dust and moisture from entering the accommodating cavity to contaminate the detection tube 200 or interfere with the detection signals of the detection assembly 300, thereby ensuring the accuracy and stability of the wastewater detection apparatus. Furthermore, the detection tube 200 and the detection assembly 300 are protected, which prevents damage to the detection tube 200 and the detection assembly 300, thereby prolonging the service life of the wastewater detection apparatus.

In some embodiments, as shown in FIGs. 1 to 5, the water inlet 111 and the wastewater outlet on the housing 100 can be connected in series to the wastewater pipeline, such that wastewater can enter the detection tube 200 inside the accommodating cavity via the water inlet 111. Meanwhile, the transmitter 310 and the receiver 320 of the detection assembly 300 are oppositely located on both sides of the detection tube 200, so as to detect the wastewater flowing through the detection tube 200 and acquire the cleanliness of the wastewater.

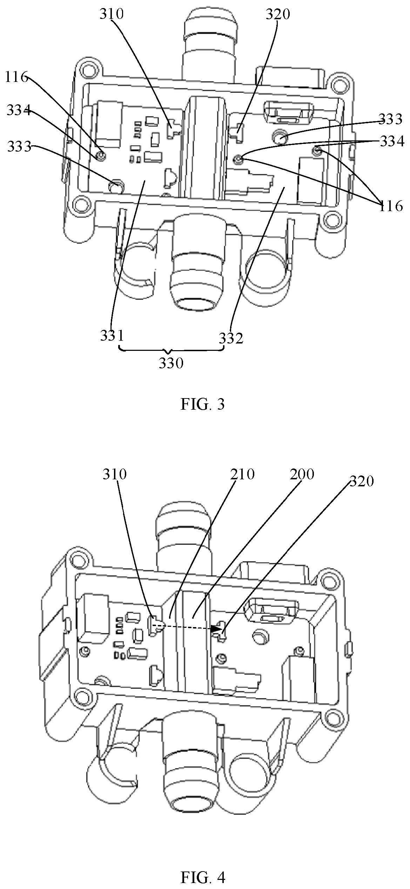

In some embodiments, as shown in FIGs. 3 to 5, the detection assembly 300 is a light detection assembly 300, an electrical signal detection assembly 300, an ultrasonic detection assembly 300, or other detection assemblies 300 that can be configured to detect parameters such as the turbidity of the wastewater.

As an optional embodiment, as shown in FIGs. 1 to 5, the detection assembly 300 is a light detection assembly 300. At least two opposite sides of the detection tube 200 are provided with light-transmitting surfaces 210. The transmitter 310 and the receiver 320 correspond to the light-transmitting surfaces 210.

In some embodiments, as shown in FIGs. 1 to 5, light-transmitting surfaces 210 are provided on at least two opposite sides of the detection tube 200. The transmitter 310 and the receiver 320 are oppositely arranged on both sides of the detection tube 200, such that the transmitter 310 and the receiver 320 correspond to the light-transmitting surfaces 210, respectively. Meanwhile, the detection assembly 300 is a light detection assembly 300. That is, the transmitter 310 emits detection light, and the receiver 320 is configured to receive the detection light after the detection light passes through the detection tube 200. When the detection light passes through the detection tube 200, due to the influence of wastewater in the detection tube 200, part of the detection light will be scattered and have its propagation direction altered. The receiver 320 can only receive part of the detection light emitted by the transmitter 310. The detection assembly 300 can acquire the impurity content in the wastewater inside the detection tube 200 based on the amount of light loss received by the receiver 320, to determine the cleanliness of the wastewater.

In some embodiments, at least two sets of transmitters 310 and receivers 320 can be provided in the detection assembly 300, such that one of the two sets of transmitters 310 and receivers 320 is configured for detection, and the other set of the two sets of transmitters 310 and receivers 320 is configured as a blank reference.

Since the detection assembly 300 is a light detection assembly 300, and the light-transmitting surfaces 210 of the detection tube 200 are greatly affected by the environment, in the embodiments of the present disclosure, the detection tube 200 and the detection assembly 300 are arranged in a sealed accommodating cavity, such that dust, moisture, and the like can be prevented from entering the accommodating cavity to contaminate the detection tube 200 or interfere with the detection assembly 300, thereby improving the detection accuracy of the wastewater detection apparatus.

As an optional embodiment, as shown in FIGs. 3 to 5, at least the light-transmitting surfaces 210 of the detection tube 200 are perpendicular to the detection light emitted by the light detection assembly 300.

In some embodiments, as shown in FIGs. 3 to 5, by at least ensuring that the light-transmitting surfaces 210 of the detection tube 200 are perpendicular to the detection light emitted by the detection assembly 300, it is possible to, to a certain extent, avoid phenomena such as refraction affecting the passage path of the detection light as the detection light passes through the light-transmitting surfaces 210 of the detection tube 200. This allows the detection light remaining after passing through the detection tube 200 and the wastewater within the detection tube 200 to basically maintain an original passage path, such that the remaining detection light can be received and detected by the receiver 320. Moreover, the impurity content in the wastewater in the detection tube 200 is acquired based on the intensity of the received detection light, to determine the cleanliness of the wastewater.

As an optional embodiment, as shown in FIGs. 3 to 6, the detection tube 200 is made of a light-transmitting material.

In some embodiments, as shown in FIGs. 3 to 6, the detection tube 200 may be made of a light-transmitting material. For example, the detection tube 200 may be made of quartz glass, transparent plastic, or the like, such that the detection tube 200 is provided with a light-transmitting surface 210 for detection. The detection tube 200 may be manufactured separately with respect to the housing 100. The separately manufactured detection tube 200 is arranged in the accommodating cavity, such that both ends of the detection tube are in communication with the water inlet 111 and the wastewater outlet, respectively. In this way, wastewater, when flowing in the accommodating cavity, only passes through the detection tube 200, without affecting the detection assembly 300 in the accommodating cavity. Meanwhile, optionally, the housing 100 may be, based on design requirements, made of a metal material or manufactured separately from a plastic material. For example, the housing 100 is separately manufactured by an injection molding process, so as to form, within the housing 100, an accommodating cavity that is provided with a water inlet 111, a water outlet 112, and a wiring hole 113, such that the detection tube 200 and the detection assembly 300 can be arranged within the accommodating cavity of the housing 100.

In some embodiments, the sealing performance of the detection tube 200 in communication with the water inlet 111 and the wastewater outlet can be improved by sealing materials such as sealant, thereby preventing leakage of wastewater at the water inlet 111 and the water outlet 112.

In some other embodiments, for example, when there is no requirement for the light transmittance of the detection tube 200 during the detection process of the detection assembly 300, the detection tube 200 itself may also have light-shielding properties.

As an optional embodiment, the housing 100 and the detection tube 200 are formed by two-color injection molding, the housing 100 is made of a light-shielding injection molding material, and the detection tube 200 is made of a light-transmitting injection molding material; or, the housing 100 includes a housing 100 body and a light-shielding layer provided on the housing 100 body, the housing 100 body and the detection tube 200 are integrally formed by injection molding, and the housing 100 body and the detection tube 200 are made of a light-transmitting injection molding material.

In some embodiments, the housing 100 and the detection tube 200 may be manufactured by a two-color injection molding process; the housing 100 is made of a light-shielding injection molding material, and the detection tube 200 is made of a light-transmitting injection molding material. After being manufactured by the two-color injection molding process, the detection tube 200 as a whole possesses light-transmitting properties, such that the detection tube 200 can meet the light transmittance requirements of the light detection assembly 300; the housing 100 as a whole possesses light-shielding properties, thereby preventing ambient light from entering the accommodating cavity within the housing 100 and avoiding interference from ambient light with the detection results of the light detection assembly 300.

In some other embodiments, the housing 100 body and the detection tube 200 may be integrally manufactured by an injection molding process. The housing 100 body and the detection tube 200 may be made of a light-transmitting injection molding material. After being integrally manufactured by the injection molding process, the housing 100 body and the detection tube 200 as a whole possess light-transmitting properties. Optionally, to prevent ambient light from entering the accommodating cavity within the housing 100 and interfering with the detection results of the light detection assembly 300, a light-shielding layer may be provided on the housing 100 body to prevent ambient light from entering the accommodating cavity within the housing 100.

In the above embodiments, the housing 100 may be formed by injection molding using PET (polyester resin) material.

As an optional embodiment, as shown in FIGs. 1 to 6, the water inlet 111 is provided with a water inlet connector 1111, and the water outlet 112 is provided with a water outlet connector 1121.

In some embodiments, as shown in FIGs. 1 to 6, the water inlet 111 is correspondingly provided with a water inlet connector 1111, and the water outlet 112 is correspondingly provided with a water outlet connector 1121, such that the water inlet connector 1111 and the water outlet connector 1121 protrude relative to the housing 100, thereby facilitating connection with a wastewater pipe.

In some embodiments, as shown in FIGs. 1 to 6, the water inlet connector 1111 may be provided with an anti-detachment convex ring protruding relative to the outer wall of the water inlet connector 1111, such that after the water inlet connector 1111 is connected to the wastewater pipe, the wastewater pipe is prevented from detaching from the water inlet connector 1111. Similarly, the water outlet connector 1121 may be provided with an anti-detachment convex ring protruding relative to the outer wall of the water outlet connector 1121, such that after the water outlet connector 1121 is connected to the wastewater pipe, the wastewater pipe is prevented from detaching from the water outlet connector 1121.

As an optional embodiment, as shown in FIGs. 1 to 6, a connecting part 114 is provided on the outer side of the housing 100, and the connecting part 114 is configured for connection with the cleaning device.

In some embodiments, as shown in FIGs. 1 to 6, the housing 100 may be provided with a connecting part 114, with the connecting part 114 being configured for connection with the cleaning device, such that the wastewater detection apparatus can be fixed in the cleaning device. For example, the connecting part 114 protrudes relative to the housing 100, and a through hole is formed in the connecting part 114. A connecting member 122, such as a screw, passes through the through hole and is threadedly connected to the cleaning device, such that the housing 100 can be fixed in the cleaning device, thereby fixing the entire wastewater detection apparatus in the cleaning device.

As an optional embodiment, as shown in FIGs. 1 to 8, the housing 100 includes a first shell 110 and a second shell 120, the second shell 120 being fastened onto the first shell 110 to enclose and form an accommodating cavity.

In some embodiments, as shown in FIGs. 1 to 8, the housing 100 may include a first shell 110 and a second shell 120. By fastening the second shell 120 onto the first shell 110, an accommodating cavity can be enclosed and formed. The first shell 110 may serve as a main shell, with the detection tube 200 and the detection assembly 300 being arranged and relatively fixed within the first shell 110, while the second shell 120 serves as a housing cover fastened onto the first shell 110.

In some embodiments, as shown in FIGs. 1 to 8, when the second shell 120 serves as the housing cover, the second shell may be plate-shaped. By arranging the plate-shaped second shell 120 as a cover on the first shell 110, the second shell can form a sealed accommodating cavity with the first shell 110.

In some embodiments, the shape and volume of the first shell 110 may be set according to the dimensional specifications of the detection tube 200 and the detection assembly 300, such that the detection tube 200 and the detection assembly 300 can be placed within the accommodating cavity mainly formed by the first shell 110.

In some embodiments, as shown for example in FIGs. 1 to 8, a threaded hole may be provided on the first shell 110, and a corresponding through hole may be formed in the second shell 120. By using a screw as the connecting member 122 to pass through the through hole and connect with the threaded hole, the second shell 120 can be fastened and fixed onto the first shell 110. For another example, a through hole may be provided on the first shell 110, and a corresponding threaded hole may be formed in the second shell 120. A screw passes through the through hole to be connected to the threaded hole, such that the second shell 120 can be fastened and fixed together with the first shell 110. Optionally, as shown in FIGs. 1, 2, and 7, a connecting washer 123 may be provided under the connecting member 122 to enhance the fastening connection effect of the connecting member 122 on the first shell 110 and the second shell 120, and to prevent the connecting member 122 from rotating and becoming detached.

As an optional embodiment, as shown in FIGs. 1 to 8, a sealing rubber sleeve 1131 is provided at the wiring hole 113; a sealing gasket 121 or a sealing ring is provided on one side of the second shell 120 facing the first shell 110.

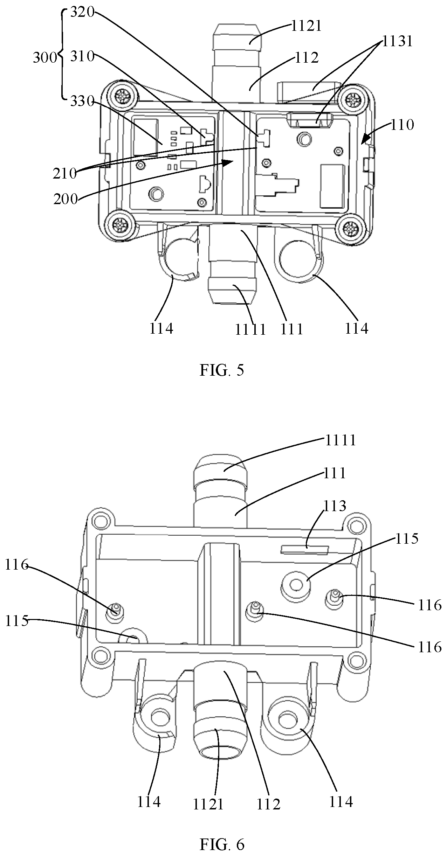

In some embodiments, as shown in FIGs. 3 and 6, a sealing rubber sleeve 1131 is provided at the wiring hole 113. When the wire harness passes through the wiring hole 113, the sealing rubber sleeve 1131 can seal the gap between the wiring hole 113 and the wire harness, thereby preventing dust, moisture, and the like from entering the accommodating cavity of the housing 100 through the wiring hole 113.

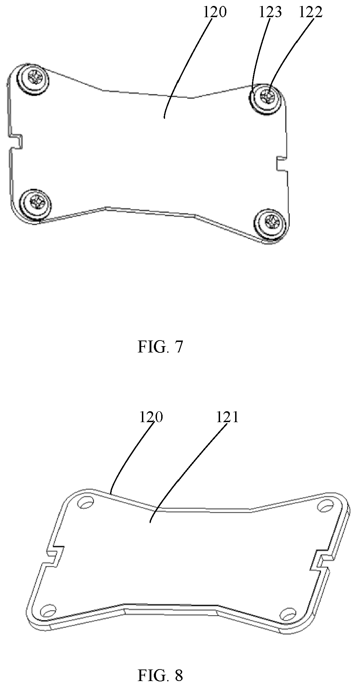

In some embodiments, as shown in FIGs. 1 and 8, a sealing gasket 121 or a sealing ring is provided on one side of the second shell 120 facing the first shell 110. When the second shell 120 is fastened onto the first shell 110, the sealing gasket 121 or the sealing ring is located on the contact position between the second shell 120 and the first shell 110. When the second shell 120 and the first shell 110 are connected by fixing members such as screws, the second shell 120 can be pressed against the first shell 110, and the sealing gasket 121 or the sealing ring seals the gap between the first shell 110 and the second shell 120, thereby preventing dust, moisture, and the like from entering the accommodating cavity of the housing 100 through the wiring hole 113.

In the wastewater detection apparatus of the present disclosure, by providing sealing structures such as the sealing rubber sleeve 1131, the sealing gasket 121, and the sealing ring, the gaps or through holes on the housing 100 can be sealed, allowing the accommodating cavity to remain clean and dry. This ensures that the outer side of the detection tube 200 remains clean and that the detection assembly 300 remains clean and dry. While ensuring the detection performance of the detection assembly 300, the detection tube 200 and the detection assembly 300 can also be protected, thereby extending the service life of the detection assembly 300 and the wastewater detection apparatus.

In some embodiments, the sealing gasket 121 may include a foam layer. Sealing between the first shell 110 and the second shell 120 is achieved by the deformation of the foam layer.

As an optional embodiment, as shown in FIGs. 3 to 6, a fixing part 115 and/or a positioning part 116 is provided on the inner side of the housing 100. The fixing part 115 is configured to fix the circuit board 330, and the positioning part 116 is configured to position the circuit board 330.

In some embodiments, as shown in FIGs. 3 to 6, the inner side of the housing 100 may be provided with a fixing part 115. The circuit board 330 can be fixed in the accommodating cavity by the fixing part 115, such that the position of the circuit board 330 in the accommodating cavity is fixed. For example, the fixing part 115 is a threaded hole provided at the bottom of the first shell 110. Correspondingly, a fixing hole 333 is formed on the circuit board 330, allowing a connecting member 122, such as a screw, to pass through the fixing hole 333 on the circuit board 330 to be connected to the threaded hole, such that the circuit board 330 can be fixed at the bottom of the first shell 110.

In some embodiments, as shown in FIGs. 3 to 6, the transmitter 310 and the receiver 320 are generally arranged on the circuit board 330. Therefore, there is a relatively high requirement for the position at which the circuit board 330 is arranged. In the wastewater detection apparatus of the present disclosure, a positioning part 116 may be provided on the inner side of the housing 100 to position the circuit board 330 via the positioning part 116, thereby improving the precision of the installation positions of the transmitter 310 and the receiver 320 within the accommodating cavity. For example, the positioning part 116 is a positioning post arranged at the bottom of the first shell 110 and protruding relative to the bottom of the first shell 110. Correspondingly, a positioning hole 334 is formed on the circuit board 330. After aligning the positioning hole 334 on the circuit board 330 with the positioning post, the circuit board 330 can be arranged at the bottom of the first shell 110.

As an optional embodiment, as shown in FIGs. 3 to 5, the circuit board 330 includes a circuit substrate, as well as a transmitting-end circuit module 331 and a receiving-end circuit module 332 that are arranged on the circuit substrate. The transmitting-end circuit module 331 includes a transmitter 310, and the receiving-end circuit module 332 includes a receiver 320. The transmitting-end circuit module 331 and the receiving-end circuit module 332 are arranged on the same circuit substrate or arranged respectively on two circuit substrates.

In some embodiments, as shown in FIGs. 3 to 5, in the detection assembly 300, the transmitter 310 is arranged in the transmitting-end circuit module 331, and the receiver 320 may be arranged in the receiving-end circuit module 332. Meanwhile, the transmitting-end circuit module 331 and the receiving-end circuit module 332 may be respectively arranged on two circuit substrates. For example, as shown in FIGs. 3 to 5, the two circuit substrates may be arranged on opposite sides of the detection tube 200, such that the transmitter 310 and the receiver 320 are located on opposite sides relative to the detection tube 200. In the embodiments, the two substrates can be easily installed on both sides of the detection tube 200 in the accommodating cavity, and there are no special requirements regarding whether there is a gap between the detection tube 200 and the first shell 110 or the size of such a gap.

In some other embodiments, in the detection assembly 300, the transmitter 310 is arranged in the transmitting-end circuit module 331, and the receiver 320 may be arranged in the receiving-end circuit module 332. Meanwhile, the transmitting-end circuit module 331 and the receiving-end circuit module 332 are both arranged on the same circuit substrate. The entire circuit substrate may be placed at the bottom of the first shell 110, with the transmitter 310 and the receiver 320 located on opposite sides of the detection tube 200. Since the transmitting-end circuit module 331 and the receiving-end circuit module 332 are arranged on the same circuit substrate, the overall size of the circuit substrate is relatively large, which results in a relatively complex installation of the detection assembly 300. In the embodiments, the detection tube 200 may be manufactured separately, and the installation sequence of the detection tube 200 and the detection assembly 300 is adjusted, such that the detection assembly 300 is first installed in the accommodating cavity, and then the detection tube 200 is installed in the accommodating cavity.

Based on the same inventive concept, the embodiments of the present disclosure further provide a cleaning device. The cleaning device includes the wastewater detection apparatus described above.

Since the cleaning device provided in the present disclosure includes the wastewater detection apparatus of the above technical solutions, the cleaning device provided in the present disclosure possesses all of the advantageous effects of the wastewater detection apparatus described above, which will not be repeated here.

In some embodiments, the cleaning device may be a handheld cleaning device, a cleaning robot, a cleaning base station, or other devices that generate wastewater during the cleaning process.

In the present disclosure, unless otherwise clearly specified and defined, a first feature being "on" or "beneath" a second feature may include that the first and second features are in direct contact and that the first and second features are not in direct contact but are in contact via an additional feature therebetween. Moreover, a first feature being "on", "over", and "above" a second feature includes that the first feature is right above or obliquely above the second feature, or simply means that the first feature is at a horizontally higher position than the second feature. A first feature being "under", "beneath", and "below" a second feature includes that the first feature is right below or obliquely below the second feature, or simply means that the first feature is at a horizontally lower position than the second feature.

In the present disclosure, unless otherwise clearly specified and defined, the terms "connect", "fix", and the like should be interpreted in their broad senses. For example, "fix" may be fixed connection, detachable connection, or integration; mechanical connection or electrical connection; direct connection or indirect connection via an intermediate; or internal communication between two elements or interaction between two elements, unless otherwise clearly defined. For those of ordinary skill in the art, the specific meanings of the above terms in the present disclosure can be understood according to specific conditions.

Although the embodiments of the present disclosure have been illustrated and described, it can be understood by those of ordinary skill in the art that various changes, modifications, replacements, and variations can be made to these embodiments without departing from the principle and purpose of the present disclosure, and the scope of the present disclosure is defined by the claims and equivalents thereof.

## Claims

1. A wastewater detection apparatus, comprising:
a housing, the housing being provided with an accommodating cavity therein, and the housing being provided with a water inlet, a water outlet, and a wiring hole that are in communication with the accommodating cavity;
a detection tube, the detection tube being arranged in the accommodating cavity, and two ends of the detection tube being in communication with the water inlet and the water outlet, respectively; and
a detection assembly, the detection assembly being arranged in the accommodating cavity, and the detection assembly comprising a transmitter, a receiver, a circuit board connected to the transmitter and the receiver, and a wire harness connected to the circuit board, the transmitter and the receiver are arranged oppositely on two sides of the detection tube, and the wire harness extends to outside of the housing through the wiring hole.

2. The wastewater detection apparatus according to claim 1, wherein the detection assembly is a light detection assembly, at least two opposite sides of the detection tube are provided with light-transmitting surfaces, and the transmitter and the receiver correspond to the light-transmitting surfaces.

3. The wastewater detection apparatus according to claim 2, wherein at least the light-transmitting surfaces of the detection tube are perpendicular to detection light emitted by the light detection assembly.

4. The wastewater detection apparatus according to claim 2, wherein the detection tube is manufactured from a light-transmitting material.

5. The wastewater detection apparatus according to claim 2, wherein the housing and the detection tube are formed by two-color injection molding, the housing is made of a light-shielding injection molding material, and the detection tube is made of a light-transmitting injection molding material; or, the housing comprises a housing body and a light-shielding layer provided on the housing body, the housing body and the detection tube are integrally formed by injection molding, and the housing body and the detection tube are made of a light-transmitting injection molding material.

6. The wastewater detection apparatus according to any one of claims 1 to 5, wherein the water inlet is provided with a water inlet connector, and the water outlet is provided with a water outlet connector.

7. The wastewater detection apparatus according to any one of claims 1 to 6, wherein a connecting part is provided on an outer side of the housing, and the connecting part is configured for connection with a cleaning device.

8. The wastewater detection apparatus according to any one of claims 1 to 7, wherein the housing comprises a first shell and a second shell, and the second shell is fastened onto the first shell to enclose and form the accommodating cavity.

9. The wastewater detection apparatus according to claim 8, wherein the wiring hole is provided with a sealing rubber sleeve; a sealing gasket or a sealing ring is provided on one side of the second shell facing the first shell.

10. The wastewater detection apparatus according to claim 5, wherein a fixing part and/or a positioning part is provided on an inner side of the housing; the fixing part is configured to fix the circuit board, and the positioning part is configured to position the circuit board.

11. The wastewater detection apparatus according to any one of claims 1 to 10, wherein the circuit board comprises a circuit substrate, as well as a transmitting-end circuit module and a receiving-end circuit module that are arranged on the circuit substrate; the transmitting-end circuit module comprises the transmitter, the receiving-end circuit module comprises the receiver, and the transmitting-end circuit module and the receiving-end circuit module are arranged on the same circuit substrate or arranged respectively on two circuit substrates.

12. A cleaning device, comprising the wastewater detection apparatus according to any one of claims 1 to 11.
